# EUROPEAN PATENT SPECIFICATION

(11) **EP 2 801 046 B1**
(45) Date of publication and mention of the grant of the patent: **15.03.2017**
(21) Application number: 13702929.4
(22) Date of filing: 07.01.2013
(51) Int. Cl.: G06F 19/16, G01N 33/94

(54) **METHOD AND SYSTEM FOR INDENTIFYING COMPOUNDS THAT BIND AND/OR ACTIVATE A TARGET OPIOID RECEPTOR IN A PH-DEPENDENT MANNER**
VERFAHREN UND SYSTEM ZUR IDENTIFIKATION VON VERBINDUNGEN, DIE EINEN ZIELOPIOIDREZEPTOR ABHÄNGIG VOM PH-WERT BINDEN UND VORZUGSWEISE AKTIVIEREN
PROCÉDÉ ET SYSTÈME D'IDENTIFICATION DE COMPOSÉS SE LIANT ET ACTIVANT DE PRÉFÉRENCE UN RÉCEPTEUR OPIOÏDE CIBLE DE MANIÈRE DÉPENDANTE DU PH

(30) Priority: 06.01.2012 EP 12150376
(43) Date of publication of application: 12.11.2014
(73) Proprietor: Stein, Christoph, 10825 Berlin (DE)
(72) Inventor: STEIN, Christoph, 10825 Berlin (DE); WEBER, Marcus, 12045 Berlin (DE); SCHARKOI, Olga, 12107 Berlin (DE); DEUFLHARD, Peter, 14199 Berlin (DE)
(74) Representative: Hertin und Partner Rechts- und Patentanwälte PartG mbB
(86) International application number: PCT/EP2013/050156
(87) International publication number: WO 2013/102681

(56) References cited:
- WO-A2-2004/079329
- US-A1- 2008 070 964
- WU G ET AL: "Opioid ligand/receptor interactions as revealed from analysis of the pH dependence of binding", ABSTRACTS OF THE ANNUAL MEETING OF THE SOCIETY FOR NEUROSCIENCE, SOCIETY FOR NEUROSCIENCE, WASHINGTON, DC, US, vol. 23, 1 January 1997 (1997-01-01), page 1206, XP009157162, ISSN: 0190-5295
- WILLIAM G REIFENRATH ET AL: "Synthesis and biological activity of fluoroalkylamine derivatives of narcotic analgesics", JOURNAL OF MEDICINAL CHEMISTRY, AMERICAN CHEMICAL SOCIETY, US, vol. 23, no. 9, 1 September 1980 (1980-09-01), pages 985-990, XP009157382, ISSN: 0022-2623, DOI: 10.1021/JM00183A005
- KOVÁCS Z ET AL: "Site-specific acid-base properties of pholcodine and related compounds", ANALYTICAL AND BIOANALYTICAL CHEMISTRY, SPRINGER, BERLIN, DE, vol. 386, no. 6, 27 September 2006 (2006-09-27), pages 1709-1716, XP019458089, ISSN: 1618-2650, DOI: 10.1007/S00216-006-0760-3
- MAHESH DR ET AL: 'PHARMACOLOGY OF OPIOIDS - PART 1 ANAESTHESIA TUTORIAL OF THE WEEK 64 12TH AUGUST 2007' INTERNET ATICLE, [Online] 12 August 2007, pages 1 - 7, XP055212867 Retrieved from the Internet: <URL:http://www.aagbi.org/sites/default/fil es/64-Pharmacology-of-Opioids-part-I.pdf> [retrieved on 2015-09-11]

## Description

The invention relates to a method for identifying candidate compounds that bind and preferably activate a target opioid receptor in a pH-dependent manner. The present invention relates also to the influence of an inflamed (acidic) environment on opioid receptor-ligand interactions and provides a method for identifying candidate compounds that bind and preferably activate a target opioid receptor in a pH-dependent manner, such that opioid receptors are exclusively or preferentially activated in injured or inflamed tissue. Such compounds are intended for selectively modulating peripheral opioid receptors without causing side effects via opioid receptors in physiological (neutral) environments (for example in the gut or central nervous system). The invention thereby enables the identification of effective analgesic compounds that do not exhibit the side effects commonly associated with conventional natural or synthetic opioid analgesics.

### BACKGROUND OF THE INVENTION

The use of currently available analgesics is limited by major adverse side effects. Non-steroidal analgesics ("NSAIDs") cause gastrointestinal ulcers, bleeding and cardiovascular complications, and opioid drugs produce drowsiness, nausea, respiratory depression, constipation, tolerance or addiction. Following painful peripheral tissue injury and inflammation, opioid receptors on peripheral terminals of primary sensory neurons are upregulated, their G-protein coupling, signaling and recycling is enhanced, and their activation results in potent inhibition of neuronal excitability and analgesia. The augmented intracellular signaling suggests conformational alterations of opioid receptors or ligands in the inflamed environment. Systemically applied conventional opioid agonists (e.g. morphine) activate both peripheral and central (brain) opioid receptors. Activation of opioid receptors in physiological (neutral) environments (e.g. brain, gut) leads to severe side effects, leaving a significant need in the field of pain treatment for compounds that work preferably selectively on peripheral receptors in injured (inflamed) tissue.

Inflammation, accompanied by tissue acidosis, is an essential component of a large group of painful syndromes (Stein, C. et al. Brain Res Rev 60, 90-113 (2009)), including arthritis, skin inflammation, inflammatory back pain, headache (certain types of neurogenic migraine), inflammatory lesions of the nervous system (neuropathic pain), cancer pain, disorders of the immune system (HIV/AIDS, multiple sclerosis), traumatic and postoperative pain. Currently available analgesic drugs are limited by unacceptable side effects such as the central actions of opioids (e.g. sedation, respiratory depression, nausea, addiction, tolerance), the intestinal effects of opioids (constipation, ileus), the gastrointestinal and cardiovascular effects of cyclooxygenase (COX) inhibitors (e.g. bleeding, ulcers, thrombo-embolic complications) and the adverse effects of anticonvulsants and antidepressants (e.g. sedation, ataxia, arrhythmias, coronary vasoconstriction). Therefore, there is need of development of new generations and formulations of analgesics which are devoid of these side effects but retain clinical efficacy.

This can be achieved by targeting opioid receptors on peripheral terminals of dorsal root ganglion (DRG) neurons (also called nociceptors or primary sensory neurons) through the local application of exogenous, or the release of endogenous opioids within injured tissue. Moreover, a large proportion (50 - 100 %) of the antinociceptive effects produced by systemically administered opioids can be mediated by such peripheral opioid receptors, and opioid agonists that do not readily enter the central nervous system (CNS) can have the same analgesic efficacy as conventional opioids (Stein, C. et al. Pharmacol Rev 2011, 63:860-881, Craft, R.M., et al., J Pharmacol ExpTher275, 1535-42 (1995), amongst others). In addition, peripherally acting opioids have been shown to reduce inflammation by modulating proinflammatory mediators, edema, plasma extravasation and other parameters (Stein, C. et al. Curr Pharm Design 2012; 18(37):6053-6069). However, despite the fact that peripherally acting opioids have been shown to reduce inflammation, leading to potential success in pain relief strategies regarding modulation of peripheral receptors, few methods have been disclosed for effectively targeting selectively peripheral opioid receptors within injured tissue without effects on either the gut or CNS.

Opioid pharmacology commands a huge armamentarium of non-peptidic and peptidic opioid receptor ligands. The most thoroughly studied include the alkaloid morphine, the piperidine fentanyl, and the enkephalin derivative (D-Ala²N-MePhe⁴Gly⁵-ol)-enkephalin (DAMGO). All of these bind to the mu-receptor, the most important receptor type for the mediation of analgesic effects in animals and humans (Zöllner, C. and Stein, C. Handbook of Experimental Pharmacology (Vol. 177) Analgesia (2007)). Chemical modification of such compounds has been accomplished manifold and has produced highly selective ligands for each receptor type as well as agonists that do not enter the CNS. While the latter can induce antinociception without central side effects, they are still likely to activate opioid receptors in the physiological (neutral) environment within the submucosal tissue of the gut (when applied orally or systemically), which commonly results in the occurrence of constipation or vomiting. Therefore, it is necessary to develop opioid receptor ligands with a particular focus on specific activity in the environment of damaged (inflamed) tissue.

Fentanyl is one example (among others e.g. morphine, codeine etc.) of a potent synthetic opioid ("narcotic") analgesic with a rapid onset and short duration of action. It is a potent agonist at mu-opioid receptors. Historically it has been used to treat chronic and breakthrough pain and is commonly used before, during and after procedures as a pain reliever as well as an anaesthetic. However, fentanyl (and others, e.g. morphine, codeine etc.) exhibits significant side effects, such as nausea, vomiting, constipation, dry mouth, somnolence, confusion, hypoventilation, apnoea, tolerance and addiction, in addition to abdominal pain, headache, fatigue, weight loss, dizziness, nervousness, hallucination, anxiety, depression, flu-like symptoms, dyspepsia (indigestion), and urinary retention, which renders fentanyl (and others, e.g. morphine, codeine etc.) in many cases unusable, especially when delivered systemically to a subject in pain. There is a need in the art to develop opioid derivatives that do not exhibit the side effects commonly associated with fentanyl (and others, e.g. morphine, codeine etc.). Many of these side effects are thought to be associated with the effect of fentanyl on the CNS.

The mechanisms underlying the peripheral antinociceptive effects of opioids have been investigated in animals and humans (Stein, C., et al., Nat Med 9, 1003-8 (2003); Pharmacol Rev 2011, 63:860-881). To this end, Freund's adjuvant (CFA)-induced hind paw inflammation in rodents has been studied, in addition to the peripheral application of small, systemically inactive doses of morphine in patients undergoing surgery or suffering from chronic arthritis. In several controlled clinical studies the inventors and others have shown that in-traarticular morphine produces pain relief of similar efficacy to local anesthetics or steroids without systemic or local side effects. Such effects are apparently mediated by opioid receptors localized on peripheral terminals of DRG neurons. The activation of these receptors reduces neuronal excitability, nociceptive impulse propagation and proinflammatory neuropeptide release. In particular, it has been shown that opioid agonists inhibit the TRPV1 ion channel, which is preferentially expressed in DRG neurons and is activated by the pungent compound capsaicin, protons and other stimuli. The inhibition occurs via opioid receptor-coupled G_{i/o} proteins and the cAMP pathway. Furthermore, it has been shown that peripherally mediated opioid antinociception is particularly prominent within inflamed tissue, and that its efficacy increases with the duration of the inflammatory process. Underlying mechanisms include upregulation of synthesis, peripherally directed axonal transport and G-protein coupling of opioid receptors in DRG neurons.

Importantly, pH-values as low as 4-5 have been measured in painful inflammation (Reeh, P.W. & Steen, K.H., Prog Brain Res 113, 143-51 (1996), Woo, Y.C., et al., Anesthesiology 101, 468-75 (2004). This significant change in pH in inflamed tissue provides a potential mechanism according to which inflammation-specific active agents can be developed, which only exhibit an opioid receptor agonistic function in the area of damage, inflammation and/or pain.

It is known in the art the pH value of the surrounding environment influences the interaction or binding between an opioid receptor and ligand of said receptor. It has been shown previously that DAMGO and fentanyl binding is pH-dependent, but also that binding is ligand-dependent, whereby different ligands exhibit different pH-dependent properties (Wu et al. Abstracts of the annual meeting of the society for Neuroscience, vol. 23, 1997, p 1206). No clear relationship between the physical or physiochemical properties of potential ligands and their pH-dependent binding properties has been previously disclosed. For example Reifenrath et al. concluded that no association between pKa and opiate receptor binding was apparent for normeperidine derivatives (J. Med. Chem. 1980, 23, 985).

The distribution of positive charges on potentially protonated nitrogen groups in pholcodine-like compounds has been investigated and shown to be pH-dependent (Kovacs et al., Anal Bioanal Chem 2006, 386, 1709). Such studies provide, however, no insight into potential modification or identification of ligands with pH-dependent binding activities. Methods have been developed for identifying ligands that show pH-dependent binding to adenosine receptors via screening compounds with known in vitro or in vivo assays under different pH conditions (WO 2004/079329 A2). Similar approaches for opioid receptors have however been neither suggested nor disclosed in the art.

Trivedi et al. ("Pharmacology of Opioids - Anaesthesia Tutorial of the Week", August 12, 2007) describe the pKa values of various opioids, including fentanyl, and aspects of their pharmacokinetics. Ultimately, the prior art reveals no insight into either identification or utilisation of the relationships between ligand structure, their physical properties and pH-dependent binding to (and potentially activation of) opioid receptors.

In contrast to the large body of information on ligands, relatively little is known about the conformation of opioid receptors. High-resolution crystallized structures of opioid receptors have only become available recently, and only in inactive antagonist-bound conformations (Manglik et al. Nature 485, 321-326, 2012; Wu et al. Nature 485, 327-332, 2012; Granier et al. Nature 485, 400-404, 2012). Opioid receptors belong to the rhodopsin/class A subfamily of seven-transmembrane G-protein coupled receptors (GPCR). As such, it is possible to extrapolate information from other members of this subfamily such as the rhodopsin and adrenergic receptors. The crystal structures of rhodopsin, its ligand-free form opsin, the G-protein interacting conformation of opsin, an antagonist-bound adenosine receptor and of beta-adrenergic receptors have been solved. Furthermore, protonation and pH have been shown to play crucial roles in conformational changes and diffusional dynamics of rhodopsin, in the activation of rhodopsin, in the activation of beta-2-adrenergic receptors and on the secondary structure of an opioid receptor fragment. Protonation of ligands also appears to be important for the activation of opioid receptors. Additional data on opioid receptors are available from random mutagenesis and 3D modelling studies. This information provides a basis for computational homology modelling of opioid ligand-receptor interactions. Importantly however, current modelling strategies neither aim at peripheral opioid receptors nor take into account that opioid ligands and/or receptors may change their conformations in injured tissue.

The invention is therefore related to modelling of opioid receptor and ligand interaction, and testing the conformational dynamics of opioid receptor-ligand behavior at different pH conditions. The present invention is also related to the influence of an inflamed (acidic) environment on opioid receptor-ligand interactions and provides a method for identifying ligands that selectively activate opioid receptors in injured (acidic, low pH) tissue.

### SUMMARY OF THE INVENTION

In light of the prior art, the technical problem underlying the present invention is to provide a method or a system for identifying candidate compounds that possess effective analgesic function but do not exhibit side effects on either the gut or central nervous system commonly associated with conventional natural or synthetic opioid analgesics.

This problem is solved by the features of the independent claims. Preferred embodiments of the present invention are provided by the dependent claims.

Therefore, an object of the invention is to provide a method for identifying compounds that bind a target opioid receptor in a pH-dependent manner, comprising
a) modification of a base compound to produce a modified compound,
b) determination of the acid dissociation constant (pKa) of said modified compound,
c) selection of said modified compound for further processing when pKa of said modified compound is less than 7, preferably between 4 and 7, more preferably between 5 and 7, and
d) testing the binding of the modified compound in said target opioid receptor,
wherein the modified compound binds the target opioid receptor at a pH value below 7, and/or wherein steps a) to d) are simulations carried out by one or more computer programmes, and wherein step d) is carried out with the modified compound and the base compound, successively, in any order, using computer implemented methods in a three-dimensional (3D) binding pocket and/or receptor activation path model, wherein a modified compound is identified as binding a target opioid receptor in a pH-dependent manner when the binding of said target opioid receptor by the modified compound in step d) is improved or the same in comparison to the binding of said target opioid receptor by the base compound.

The method of the present invention is characterized in that step d) is carried out by computer simulation with the modified compound and base compound, successively, in any order, whereby binding and/or activation of the receptor by the modified compound in the binding pocket model (and/or receptor activation model) is compared to binding and/or activation of the receptor by the base compound in the binding pocket model (and/or receptor activation model), whereby desired modified compounds are identified when the binding and/or activation of the receptor caused by the modified compound is either improved, the same or similar, in comparison to the base compound.

The binding model and the activation path model relate preferably to the same model, but may involve different analyses in order to detect any given compound binding to and/or activating the receptor. In the examples of the present invention (modified morphine and fentanyl molecules), the binding event has been modeled and is demonstrated in full detail. The activation potency of a modified (or unmodified base compound as reference) can be measured and/or estimated by observing (thereby measuring or determining) the conformational changes of the alpha helices of the opioid-receptor model after binding of the test ligand to be analysed. The method of the invention therefore also encompasses simulation of receptor activation, which can occur on its own or in combination with analysis of binding between receptor and compound. The model is therefore capable of also measuring not only binding as such, but additionally (or separately) conformational changes in the receptor itself, in particular of the alpha helices of the receptor, during a binding event.

In a preferred embodiment the method of the present invention is characterized in that one or more of the steps a) to d) are simulations carried out by one or more computer programmes, preferably as individual software modules combined to be automatically carried out, on a computing device.

An acid dissociation constant, Ka, (also known as acidity constant, or acid-ionization constant) is a quantitative measure of the strength of an acid in solution. The acid dissociation constant of a small molecule is therefore determined by the dissociation constants of its acidic and basic groups. Due to the many orders of magnitude spanned by Ka values, a logarithmic measure of the acid dissociation constant is more commonly used in practice. The logarithmic constant, pKa, which is equal to -log10 Ka, is also referred to as the acid dissociation constant. For example, assume you have a compound HX, then HX can be understood as H+ + X-, whereby pKa = -log Ka, then Ka = [H+] x [X-]/[HX]. The present invention refers to pKa as commonly used. The pKa value of any given modified compound can be determined by various methods known to those skilled in the art, either computationally, or for example via chemical experimentation involving titration. Although pKa can be calculated using various methods, the methods of calculation provide very similar results to one another and to those measurements carried out by empirical experimentation. The specific method used in the examples of the invention should therefore not be considered a limiting feature.

In one embodiment the method of the present invention is characterized in that the identified modified and/or base compound is chemically synthesized or chemically synthesizable.

The modification of a base compound relates to any structural modification; preferably change, in the molecular and/or atomic structure. Methods for modification of chemical compounds are known to those in the art, especially to those skilled in chemistry and/or chemical synthesis of pharmaceutical compounds. A base compound can essentially be any chemical structure that can be used as a starting point for further chemical modification. Base compounds can therefore preferably be known opioids, or known opioid receptor ligands and/or agonists, or any other candidate molecule for which modified variants are to be sought. A modified compound therefore relates to any given compound that exhibits a structural modification in relation to a base compound.

In one embodiment the method of the present invention is characterized in that the modified compound and/or base compound, preferably in their physical form (as produced by chemical synthesis) are tested for opioid receptor binding, and preferably opioid receptor agonist function, using in vitro and/or in vivo validation methods. The in vivo and/or in vitro validation is a preferred aspect of the method but is not considered fundamental to the method of the invention. The binding of the modified compound in, and preferably activation of, said target opioid receptor may be carried out via simulation of binding of the modified (and/or base) compound in a three-dimensional (3D) binding pocket model for said target opioid receptor, preferably using computer implemented methods. Appropriate software to generate binding and/or activation models and subsequent testing of binding and/or activation using in silico approaches is also a preferred aspect of the invention.

In one embodiment the method of the present invention is characterized in that the in vitro validation comprises of measurement of GTPγS binding, G-protein activation, cAMP accumulation, modulation of TRVP1 currents, modulation of stimulus-evoked action potentials and/or any other suitable in vitro assay for determining opioid receptor activation by tested compounds. In one embodiment the method of the present invention is characterized in that the in vitro validation comprises use of embryonic kidney cells, preferably HEK 293 cells, sensory neurons and/or cultured dorsal root ganglion (DRG) neurons. Those skilled in the art are aware of various assays for assessing ligand-receptor interaction. For example the development of a variety of techniques based on atomic force microscopy, hydrodynamic flow, fluorescence resonance energy transfer, G-protein activation and/or dissociation, arrestin binding, modulation of cAMP production, modulation of membrane ion currents, biomembrane probes, optical tweezers, magnetic fields, flexible transducers or others allows direct experimental information of the behaviour of ligand-receptor interaction.

In one embodiment the method of the present invention is characterized in that the in vivo validation comprises the CFA-induced hindpaw inflammation assay, in vitro skin-nerve preparations, neuropathic pain models and/or any other suitable in vivo assay for determining analgesic effects of the tested compounds. For example, tissue damage, inflammation or injury of the nervous system may result in chronic inflammatory or neuropathic pain characterised by increased sensitivity to painful stimuli (hyperalgesia), the perception of innocuous stimuli as painful (allodynia) and spontaneous pain. Animal models of peripheral inflammatory or neuropathic pain are available in which the mechanisms underlying hyperalgesia and allodynia due to tissue/nerve injury or tissue/nerve inflammation can be analysed (as reviewed in Moalem, Tracey, Brain Research Reviews, 2006, 51 (2):240-2649). Various in vivo assays are available to assess injury/inflammation-related pain and possible effects of the compounds identified by the present method.

In one embodiment the method of the present invention is characterized in that the base compound is an opioid or opioid derivative or analogue, which exhibits a nitrogen atom that can be protonated or non-protonated. A positively charged nitrogen atom of a modified compound is considered as an important feature for effective interaction with the ligand-binding site of the receptor, whereby the positively charged nitrogen of the ligand (modified compound) is considered to interact with an anionic site of the receptor. A preferred embodiment of the present invention is characterised in that the anionic binding site of the binding pocket model is the aspartic acid residue 147 (Asp147) of the mu-opioid receptor.

In one embodiment the method of the present invention is characterized in that protonation of said nitrogen atom (that can be protonated or non-protonated) occurs in modified compounds with a pKa of less than 7 when at pH values less than 7.

In one embodiment the method of the present invention is characterized in that the target opioid receptor is selected from the group consisting of the mu-receptor, delta-receptor, kappa-receptor and/or subtypes. The binding of compounds identified using the method provided herein to the opioid receptors and the subsequent receptor activation as described herein, relates preferably to compounds binding the mu-opioid receptor. However, due to structural and/or functional similarities between various opioid receptors and/or signalling pathways involved in analgesic effects, the binding and receptor activation by the claimed compounds may also occur with opioid receptors in addition to the mu-receptor, such as the delta-receptor, kappa-receptor, or other related opioid receptors, such as target receptors of the family of opioid-related G-protein coupled receptors, such as the rhodopsin, opsin, chemokine, cannabinoid, dopamine, histamine, muscarinic, neurotensin, adrenergic, adenosine, beta-androgen and/or any other opioid-related receptor.

In one embodiment the method of the present invention is characterized in that the modification of the base compound is the replacement of one or more hydrogen atoms with one or more atoms of greater electronegativity compared to hydrogen, preferably with a halogen atom, such as Cl, Br, I, Fl, more preferably with a fluorine atom. Electronegativity can be deduced from the periodic table of electronegativity using the Pauling scale.

In one embodiment the method of the present invention is characterized in that the evaluation of binding of the modified or base compound in the binding pocket model is carried out using steric and/or energetic criteria measured and/or determined during simulation of binding.

Energetic criteria relate in their broadest sense to any measurement, simulation or calculation of the electrostatic interaction between electrically charged atoms or molecules or interactions between a pair of neutral atoms or molecules. In one embodiment the method of the present invention is characterized in that the energetic criteria for evaluation of binding and/or receptor activation of the modified or base compound in the binding pocket model and/or receptor activation path model are determined by calculation of the Leonard Jones potential and/or Coulomb interactions. In one embodiment the method of the present invention is characterized in that calculation of the energetic binding criteria for the modified and/or base compound is carried out for binding and/or receptor activation of the compound in the binding pocket model and/or receptor activation path model, and then compared to binding and/or receptor activation of the compound in water as a reference value. These measurements can be carried out using, for example, in one embodiment, the computer simulations and/or software as disclosed herein or as commonly used in the art.

Steric effects in a broad sense arise from the space occupied by each atom within a molecule. If atoms are brought too close together, there is an associated cost in energy due to overlapping electron clouds (Pauli or Born repulsion), and this may affect a molecule's preferred shape (conformation), reactivity or interaction with other molecules. In one embodiment the method of the present invention is characterized in that the steric criteria for evaluation of binding (and/or receptor activation) of the modified or base compound in the binding pocket and/or receptor activation path model are determined by measurement of the distance between reference atoms of the modified compound and the binding pocket model. This measurement can be carried out using, for example, in one embodiment, the computer simulations and/or software as disclosed herein or as commonly used in the art.

In one embodiment the method of the present invention is characterized in that the method additionally comprises identifying chemical residues (e.g. amino acids and others) within the receptor that are responsible for pH-dependent binding and/or activation of the receptor. The method preferably comprises modelling the interaction between receptor residues and the compound binding to the receptor. Through analysis of this interaction the residues of the receptor involved in pH-dependent binding can also be determined, thereby providing useful information for further modification of base compounds for future analysis.

In one embodiment the method of the present invention is characterized in that the reference atom of the modified compound is the nitrogen atom that can be protonated or non-protonated. In one embodiment the method of the present invention is characterized in that the reference atom of the binding pocket model is the aspartic acid residue 147 (Asp147) of the mu-opioid receptor.

In one embodiment the method of the present invention is characterized in that the modified compound binds and preferably activates the target opioid receptor in conditions of inflammation-associated pH in inflamed tissue, preferably at pH values between 4 and 7, such as 4, 4.5, 5, 5.5, 6 or 6.5, more preferably between 5 and 7, or 5 and 6, or any other pH value below 7. These properties of the molecules identified by the present method may be assessed by the in vitro or in vivo methods mentioned herein or by other methods known in the art.

In one embodiment the method of the present invention is characterized in that the modified compound exhibits inflammation-specific peripheral analgesic function in inflamed or injured tissues, preferably without causing central or intestinal effects.

Also disclosed is a method as described herein, further comprising formulating the identified modified compound in a pharmaceutically acceptable form. Also disclosed is a method as described herein, comprising the method as described herein and furthermore preparing the compound identified with a pharmaceutically acceptable carrier.

In one embodiment the method of the present invention is characterized in that determination of the pKa of a modified compound comprises determination of the pKa for each conformation of said modified compound and calculation of the pKa based on a weighted average of the pKa for each conformation, whereby the weighted average corresponds to the statistical weight for each conformation.

In one embodiment the method of the present invention is characterized in that simulation of binding is carried out using multiple 3D conformations of the modified compound, whereby determination of 3D conformation of a candidate compound comprises determination of potential 3D conformations of a candidate compound as meta-stable conformations in position space and determination of the statistical weights for each conformation.

In one embodiment the method of the present invention is characterized in that simulation of binding is carried out using binding path analysis. In one embodiment the method of the present invention is characterized in that simulation of receptor activation is carried out using receptor activation analysis.

In one embodiment the method of the present invention is characterized in that the 3D binding pocket model (and/or receptor activation model) is constructed by determination of size of the atoms of the binding pocket, charge of the atoms of the binding pocket and preferably charge of regions of the binding pocket model.

In one embodiment the method of the present invention is characterized in that the 3D binding pocket model (and/or receptor activation model) is constructed by determination of size of the atoms of the receptor activation path, charge of the atoms of the receptor activation path, and preferably charge of regions of the receptor activation path model.

In one embodiment the method of the present invention is characterized in that the binding pocket and/or receptor activation path model is constructed using homology modelling. Homology modelling, also known as comparative modelling refers to constructing an atomic-resolution model of a target protein from its amino acid sequence and an experimental three-dimensional structure of a related homologous protein. Homology modelling relies on the identification of one or more known protein structures likely to resemble the structure of the query sequence, and on the production of an alignment that maps residues in the query sequence to residues in the template sequence. Evolutionarily related proteins have similar sequences and naturally occurring homologous proteins have similar protein structure. It has been shown that three-dimensional protein structure is evolutionarily more conserved than would be expected on the basis of sequence conservation alone (for a review see Marti-Renom, et al. Annu Rev Biophys Biomol Struct 29: 291-325, 2000). Homology modelling may use software such as SWISS-MODEL or the like, e.g. structural bioinformatics web-servers, or protein databases (such as PDB, 2iql, 2iqo) (Fowler et al. Biochemistry 43, 15796-810, 2004).

In one embodiment the method of the present invention is characterized in that the binding pocket and/or receptor activation path model is constructed using amino acid sequences and/or crystal structures of the mu-opioid receptor, delta-opioid receptor, kappa-opioid receptor, rhodopsin, opsin, chemokine, cannabinoid, dopamine, histamine, muscarinic, neurotensin, adrenergic, adenosine, beta-androgen and/or any opioid-related receptor.

### DETAILED DESCRIPTION OF THE INVENTION

A method for identifying candidate compounds that bind and preferably activate opioid receptors in a pH-dependent manner is provided herein. Candidate compounds identified via the claimed method can then be preferably tested in transfected cell lines, cultured sensory neurons, in the *in vitro* skin-nerve preparation, in knock-in mice carrying mutant opioid receptors and/or in animal models of inflammatory or neuropathic pain.

The compounds identified and the method of the present invention transcends traditional concepts and methods of pain treatment by focusing on the specific activation of peripheral opioid receptors in injured tissues. The agonists identified via the method described herein have been demonstrated to exhibit analgesic activity in animal models of inflammatory or neuropathic pain *in vivo.* The present invention therefore relates to the influence of an inflamed (acidic) environment on opioid receptor-ligand interactions and provides a method for identifying candidate compounds that selectively bind and preferably activate a target opioid receptor in a pH-dependent manner in injured tissue.

More specifically, the method in a preferred embodiment relates to (i) simulating the conformations of opioid receptors, their binding pocket (and their G-protein binding domain and/or receptor activation path) at different pH values and protonation states; (ii) analyzing conformational changes of opioid agonists at different pH values; (iii) using transfected human embryonic kidney (HEK-293) cells, cultured primary afferent neurons and *in vitro* skin-nerve preparations to examine opioid agonist binding, GTPγS binding, cAMP accumulation and modulation of TRPV1 currents and action potentials by opioid agonists at varying pH values; (iv) analyzing receptor docking and/or receptor activation of protonated versus deprotonated opioid agonists; (v) using this information to design novel opioid agonists that selectively activate opioid receptors at acidic but not at normal pH values; and (vi) testing these compounds for antinociceptive efficacy in animal models of inflammatory and/or neuropathic pain.

The identification method of the present invention applies, in a preferred embodiment, computational methods and software for the simulation of binding processes (and/or the receptor activation path) and conformational stability of the opioid receptor and opioid ligands at different pH-values, and for the computational design of novel opioid agonists.

The inventors have developed computational methods and software for a stable and efficient analysis of conformational changes of molecular systems ("conformation dynamics"). These methods allow determination of the main conformations of a molecule with their thermodynamic weights and transition probabilities. In this context, conformations are defined as almost invariant (metastable) subsets in the conformational space, not as local minima of the energy landscape of a molecule. Conformation dynamics is the ideal method to generate a canonical ensemble simulation in a well-conditioned way including error analysis. For the simulation of ligand-receptor binding via conformation dynamics, much of the protein is held fixed, whereas the active binding site and the ligand are fully sampled. The simulation of the path of a ligand into the binding pocket of a receptor (and preferably of downstream conformational alterations influencing G-protein binding) has been developed. Binding affinities can be estimated and, thus, rational drug development is feasible. Whereas standard computer methods such as docking routines focus on the enthalpic contributions to binding affinity, conformation dynamics simulations include entropical effects of the binding process. Entropical effects are particularly important for the pH dependency of opioid receptor-ligand interactions and consequent receptor function.

The fentanyl derivatives identified by the method of the present invention function as opioid receptor agonists, which bind and activate target opioid receptors in a pH-dependent manner. The fentanyl compounds as described herein relate to examples of compounds identified by the method of the present invention and limit in no way the method itself. Other compounds with different chemical structures can be identified using the method described herein. The fluoro-substituents of the herein described fluoro-fentanyl derivatives exhibit an enhanced electronegativity when compared to hydrogen and therefore lead to an enhanced electronegativity of the fentanyl derivative molecule, thus providing the compounds with their pH-dependent receptor agonist activity. Through the fluor-modification of the fentanyl backbone the overall conformation or three-dimensional structure of the molecules remains unchanged, or only changed to a minor extent, whereas the electronegativity is significantly reduced.

The "pH-dependent binding" between the compounds as described herein and the opioid receptor relates to an enhanced binding (and/or receptor activation) at pH values less than 7, in comparison to values above 7. In a preferred embodiment the binding between compound and receptor (as well as preferably receptor activation) is enhanced proportionally with lowering pH values. Obviously at very low pH values the receptor protein may undergo significant conformational change and/or denaturation, so that the binding and activation no longer occurs in an improved manner. Therefore the binding between F-fentanyl and receptor as well as receptor activation occurs preferably at pH values of 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, or any other pH value below 7, whereby the binding and activation occurs preferably between 4 and 7, preferably between 4.5 and 6.5, most preferentially at pH values between 5 and 6. Although some binding may occur at either higher or lower pH values as explicitly provided herein, the binding and activation of the receptor (and associated pain-relief in vivo) occurs most preferentially at pH levels, which are naturally lowered to approximately 4, 4.5, 5, 5.5, 6 or 6.5, or any other pH value below 7, after injury or inflammation of tissue.

The activation of the receptor occurs in a preferred embodiment via binding of the agonist to the receptor. However, the binding of the agonist to the receptor may be either a stable or transient event, of either strong or weak interaction, such that the receptor is activated preferably by agonist-receptor physical interaction. Activation of an opioid receptor by an agonist, such as morphine of fentanyl, can cause analgesia or sedation. Activation can be assessed via various methods as described herein and those known to a skilled person.

The terms "inflammatory pain" and "inflammation-associated pain" are used interchangeably. Said inflammation-associated pain relates to any kind of pain experienced by a subject when inflammation is involved. Inflammation may be detected or recognized by various physiological effects (such as swelling, warmth, redness of tissue), cellular effects (for example leukocyte activity) or molecular effects (such as cytokine or chemokine detection, for example TNF alpha detection).

The compounds of the present invention are intended for use in the treatment of arthritis, skin inflammation, inflammatory back pain, headache (including neurogenic migraine), inflammatory lesions of the nervous system (neuropathic pain), cancer pain, disorders of the immune system (HIV/AIDS, multiple sclerosis), traumatic pain, postoperative pain, inflamed joints, dental surgery, visceral pain, bone pain, burn injury, and/or eye lesions. These disorders are intended as examples of conditions that are associated with inflammatory pain and are not intended as a limiting disclosure.

The compounds detected by the method of the present invention therefore exhibit the surprising advantage that unacceptable side effects of traditional analgesic treatments are avoided, such as the central actions of opioids (e.g. sedation, respiratory depression, nausea, addiction, tolerance), the intestinal effects of opioids (constipation, ileus), the gastrointestinal and cardiovascular effects of cyclooxygenase (COX) inhibitors (e.g. bleeding, ulcers, thrombo-embolic complications) and the adverse effects of anticonvulsants and antidepressants (e.g. sedation, ataxia, arrhythmias, coronary vasoconstriction).

### FIGURES

The invention is further described by the figures. These are not intended to limit the scope of the invention.

### Brief description of the figures

- Figure 1.: Two different sub-conformations (SC) of protonated morphine
- Figure 2.: Morphine with indexed hydrogens
- Figure 3.: Fentanyl structure
- Figure 4.: F-Fentanyl binding
- Figure 5.: Competitive binding measuring the affinity of control mu-agonists to the mureceptor
- Figure 6.: Suppression of cAMP-accumulation by F-fentanyl
- Figure 7.: Antinociceptive effects of opioid ligands in vivo after intraplantar (i.pl.) injection into the hindpaw of rats
- Figure 8.: Antinociceptive effects of opioid ligands in vivo after intravenous injection

### Detailed description of the figures

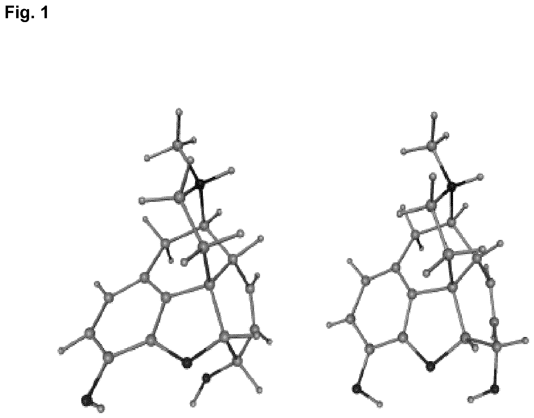

Figure 1: Two different conformations (or sub-conformations (SC)) of protonated morphine: SC1 (left) and SC2 (right) have different positions of 6-OH. Transition rates between SC1 and SC2 for protonated morphine (N+) and for deprotonated morphine (N) are shown in Tab. 1 and Tab. 2.

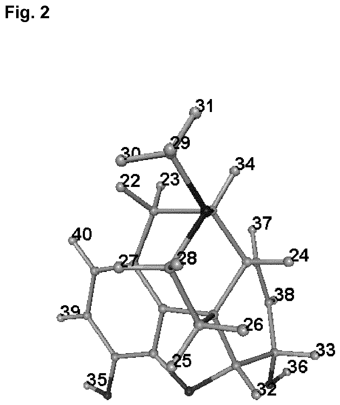

Figure 2: Morphine with indexed hydrogens. Hydrogens were replaced by fluorine and corresponding pKa-values calculated. Only results with decreased pKa-values are shown.

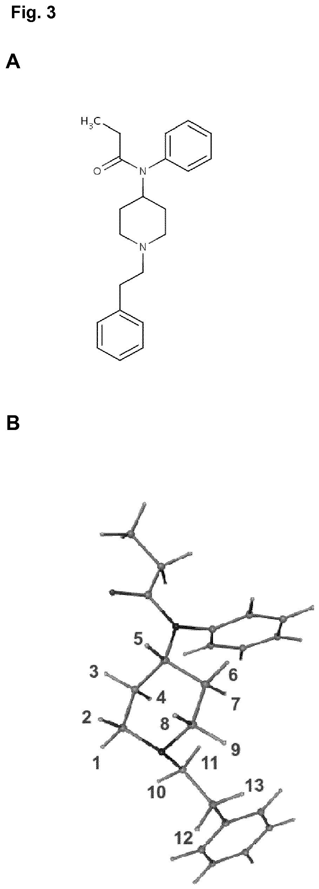

Figure 3: A) structure of fentanyl, B) fentanyl with indexed hydrogens.

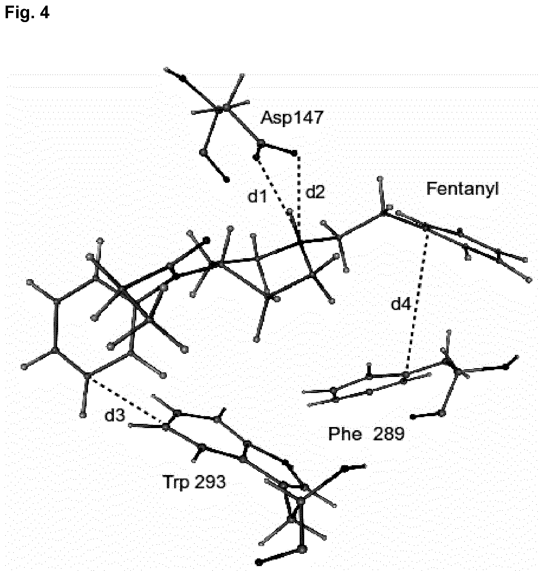

Figure 4. F-Fentanyl binding. Distances between fentanyl and Asp147, Phe 289 and Trp 293, amino acids belonging to the binding pocket of the mu-receptor are shown.

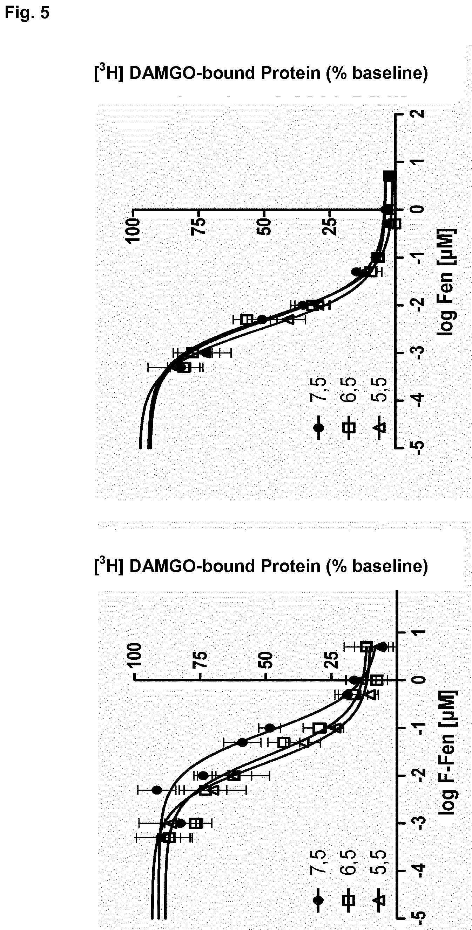

Figure 5. Competitive binding experiments measuring the affinity of control mu-agonsists to the mu-receptor.

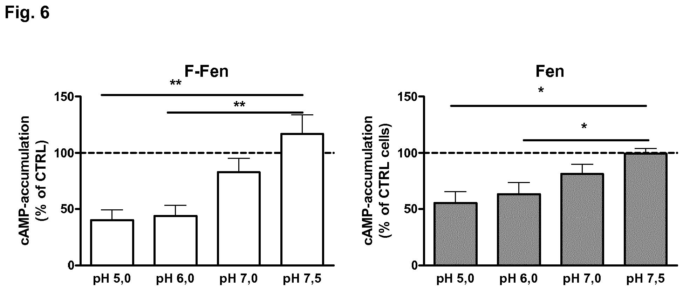

Figure 6. Suppression of FSK/IBMX-stimulated cAMP-accumulation by F-fentanyl (white) and fentanyl (grey).

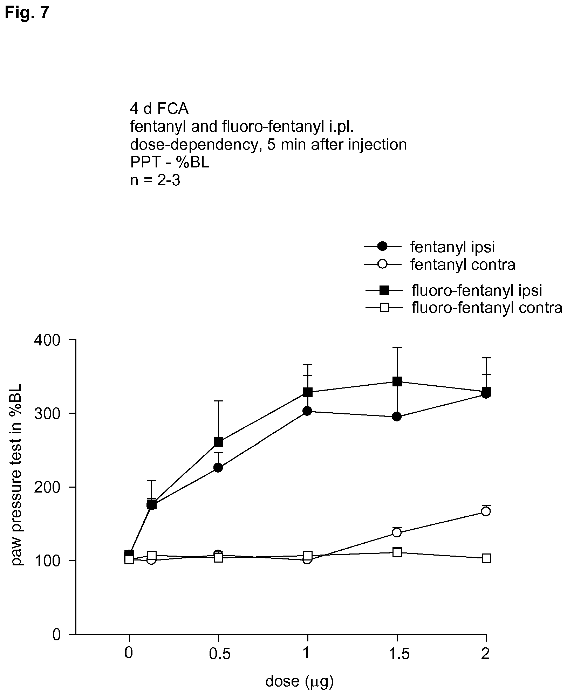

Figure 7. Antinociceptive effects of opioid ligands in vivo. Antinociceptive effects (PPT elevations in % baseline) in inflamed (ipsi) and noninflamed (contra) paws following i.pl. (intraplantar) injection of drugs in rats.

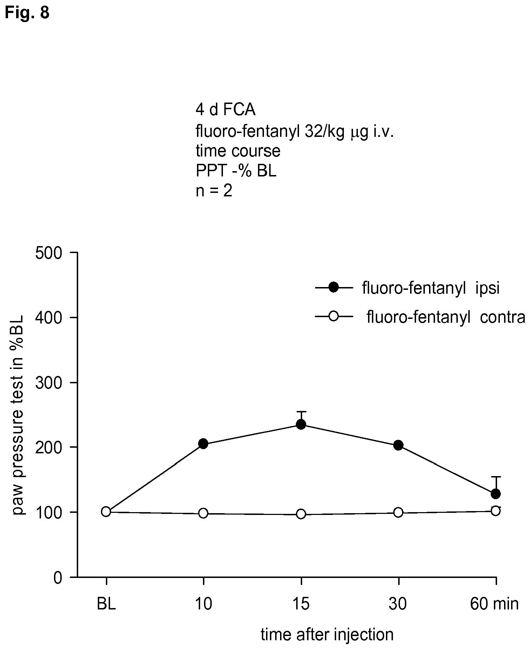

Figure 8. Antinociceptive effects of opioid ligands in vivo. Antinociceptive effects (PPT elevations in % baseline) in inflamed (ipsi) and noninflamed (contra) paws following i.v. (intravenous) injection of drugs.

### EXAMPLES

The invention is further described by the following examples. These are not intended to limit the scope of the invention. The experimental examples relate to identification and validation of candidate compounds using the method as described herein. The experiments are used to demonstrate the invention by way of example.

### Example 1: Modified Morphine

### Analysis of mu-opioid receptor conformations

Since an x-ray crystal structure of the mu-opioid-receptor was not available when the invention was conceived, the crystal structure of rhodopsin served as the basis for the modelling of the mu-opioid receptor. An accurate 3D structure model of the rat mu-opioid receptor from a protein database (PDB, 2iql, 2iqo) (Fowler et al. Biochemistry 43, 15796-810, 2004) was used. The human and rat mu-receptor share 95 % amino acid identity. The Fab fragment of the anti-morphine antibody 9B1 was also used, which exists as a crystal structure with a bound morphine molecule (PDB-ld 1Q0Y). The binding pockets have similar amino acid compositions: In 9B1, morphine's protonated nitrogen forms a salt bridge with GluH50, in the mu-receptor with Asp147.

### Conformational space of the morphine molecule at different pH values

Using software specifically designed for the purpose (ZIBgridfree, Weber, M., Scharkoi, O., FU Berlin, 2006, 2007) it was found that both protonated and deprotonated morphine have one main metastable conformation with two subconformations at 300 K (Fig. 1, Tab. 1 and Tab. 2). For protonated morphine the statistical weights are 51.85 % for subconformation 1 and 48.04 % for subconformation 2. For deprotonated morphine the statistical weights are 24.49 % for subconformation 1 and 75.50 % for subconformation 2.

| Tab. 1. Transition rates between SC1 and SC2 (N-protonated) | | |
|---|---|---|
| N+ | 1 | 2 |
| 1 | 0.55 | 0.45 |
| 2 | 0.46 | 0.54 |
| | | |

| Tab. 2. Transition rates between SC1 and SC2 (N-unprotonated) | | |
|---|---|---|
| N | 1 | 2 |
| 1 | 0.265 | 0.735 |
| 2 | 0.243 | 0.757 |

### Design of new ligands by modification of agonist pKa values

To be activated, the mu-opioid receptor preferably requires an opioid agonist with protonated N-group (Li et al, Life Sciences, vol. 65, 2, 175-185, 1999). The pKa value of the N-group of most opioids is about 8 (e.g. 8.2 for morphine, 8.4 for fentanyl). Thus, the N-group is protonated both in inflamed (pH 5-6) and in healthy (pH 7.4) tissue and opioid receptors are activated in both environments. If the pKa value of an agonist's N-group could be decreased to about 6, it would be protonated in inflamed but deprotonated in normal tissue and, therefore, it would be active only in inflamed tissue. One possibility to do this is replacement of hydrogen by fluorine atoms, which should have no major influence on the dynamic behavior of the opioid agonist. Fluorine is very electronegative and hence attracts protons and decreases the pKa-value.

The following modifications were examined by computational modelling: (i) Systematic replacement of single, pairs or triples of hydrogen atoms of an initial opioid by fluorine atoms was carried out. This represents an example of modification of a base compound as described herein according to step a), whereby the initial opioid is the base compound and the fluorinated opioid the modified compound. Calculation of the pKa-value of resulting molecules was carried out using Gaussian 09, and selection of those modified compounds with values of about 6 was subsequently carried out. This relates to the method steps b) and c) as described herein. (ii) Selection of molecules with surface, conformational space and binding characteristics most similar to the original opioid (base compound) was subsequently carried out, thereby identifying compounds with pH-specific properties but with minimally changed structures. This design concept was applied to morphine, a rigid molecule perfectly suited for sampling and illustration, yielding the results shown in Fig. 2. The conformational spaces of the new molecules (F5, F6), were then compared via ZIBgridfree. Protonated morphine, F5 and F6 have similar conformational spaces, i.e. one metastable conformation with two subconformations (Fig. 1) and similar statistical weights (Tab. 4). The electrostatic and geometrical properties of the surfaces of morphine and the modified molecules were compared via surface matching which yielded a higher score for F5 (Tab. 5).

| Tab. 3. Respective hydrogens replaced by fluorine and corresponding pKa-values. | | |
|---|---|---|
| Structure | Hydrogen Index | pKa-value |
| Morphine | --- | 8.66 |
| F1 | 29 | 4.36 |
| F2 | 30 | 3.14 |
| F3 | 31 | 3.93 |
| F4 | 27 | 3.83 |
| F5 | 28 | 6.20 |
| F6 | 34 | 6.15 |

| Tab. 4. Statistical weights of sub-conformations 1 and 2 | | |
|---|---|---|
| | 1 | 2 |
| morphine | 0.52 | 0.48 |
| F5 | 0.65 | 0.35 |
| F6 | 0.57 | 0.43 |

| Tab. 5. Surface properties | | | |
|---|---|---|---|
| Structure | Morphine | F5 | F6 |
| Score | 1 | 0.49 | 0.47 |

### Analysis of receptor docking

The binding behavior of protonated F5 and F6 was compared to that of morphine. This analysis of binding behavior relates to step d) of the method as described herein. Protonated F5 seemed to have the same stable position in the binding pocket of the mu-receptor as protonated morphine. The positions of protonated F6 and deprotonated morphine were less stable (Fig. 3). We performed a similar analysis using the Fab fragment of the anti-morphine antibody 9B1 (PDB-Id 1Q0Y) (see goal i). Replacement of one hydrogen atom of morphine by a fluorine atom did not influence its behavior in the binding pocket of 9B1 (Fig. 4).

On the basis of our simulations, F5 qualifies as a candidate compound for a drug candidate. It has a predicted pKa-value of its N-group of 6.20. The conformational space and binding behavior of protonated F5 is very similar to that of protonated morphine.

### Example 2: Modified Fentanyl

The analogous procedure to example 1 was carried out for the mu-agonist fentanyl. Through this approach fluorofentanyl (F-fentanyl) derivatives were identified and selected using the method of the present invention as candidate compounds for a drug. F-Fentanyl was subsequently synthesized and its analgesic function validated using both in vitro and in vivo approaches.

### Design of new ligands by modification and analysis of agonist pKa values

| Table 6: Modified fentanyl showing the index of replaced H molecules (with F) and pKa values | | |
|---|---|---|
| Structure | Index of replaced hydrogen | Estimated pKa |
| Fentanyl | - | 8.50 |
| F1 | 1 | -0.84 |
| F2 | 2 | 6.51 |
| F3 | 3 | 8.07 |
| F4 | 4 | 5.87 |
| F5 | 5 | -2.14 |
| F6 | 6 | 6.14 |
| F7 | 7 | 5.53 |
| F8 | 8 | -2.92 |
| F9 | 9 | 5.22 |
| F10 | 10 | 4.97 |
| F11 | 11 | -0.38 |
| F12 | 12 | 2.89 |
| F13 | 13 | -4.89 |

Table 6 shows the estimated pKa-values for fentanyl and modified fentanyl after F substitution. Indexed hydrogens were replaced by fluorine and corresponding pKa-values were calculated. Only results with decreased pKa-values (in comparison to fentanyl itself) are shown.

Due to symmetry reasons, the replacement of hydrogens with the indices 1, 2, 8 and 9 by fluorine leads to an identical structure (F1*). The same holds for the atoms with the indices 10 and 11 (F2*), and for the hydrogens with the indices 3, 4, 6 and 7 (F3*). For estimation of the pKa-value of F1*, F2* and F3* we have to calculate the mean pKa-values of the corresponding structures.

For F1*, we obtain the pKa-value of approx. 2, for F2* approx. 2.3 and for F3* approx. 6.4. Therefore, F3* qualifies as a candidate compound for a drug candidate.

### Analysis of receptor docking

For comparison of the binding behaviour, fentanyl and F3* (F7, F6, F3, F4) were simulated in the binding pocket of the mu-receptor as described above for morphine and morphine derivatives. The binding affinities were estimated (table 7).

| Table 7: Estimated binding affinities of fentanyl and F3* to the mu-receptor. We found 5 stable positions for fentanyl, one stable position for F7, one for F6, 2 for F3 and 2 for F4. | |
|---|---|
| | Delta G in kJ/mol |
| Fentanyl a | -141.22 |
| Fentanyl b | -89.04 |
| Fentanyl c | -116.09 |
| Fentanyl d | -141.94 |
| Fentanyl e | -194.34 |
| F7 | -88.96 |
| F6 | -74.84 |
| F3 a | -119.77 |
| F3 b | -77.13 |
| F4 a | -45.10 |
| F4 b | -102.87 |

Mean distances between selected atoms of the ligands and selected atoms of certain amino acids were measured (figure 4, table 8).

| Table 8: Average distances between fentanyl and modified fentanyl and Asp147, Phe 289 and Trp 293 (figure 3) in angstroms | | | | |
|---|---|---|---|---|
| | d1 | d2 | d3 | d4 |
| Fentanyl a | 2.8 | 4.7 | 3.6 | 4.8 |
| Fentanyl b | 4.8 | 2.7 | 13.6 | 8.3 |
| Fentanyl c | 3.2 | 2.8 | 10.8 | 9.7 |
| Fentanyl d | 3.3 | 2.7 | 9.9 | 9.1 |
| Fentanyl e | 3.6 | 2.7 | 4.2 | 10.4 |
| F7 | 2.8 | 4.6 | 11.8 | 9.3 |
| F6 | 3.0 | 3.3 | 6.7 | 11.7 |
| F3 a | 2.8 | 4.6 | 4.3 | 11.9 |
| F3 b | 2.8 | 4.9 | 5.2 | 13.1 |
| F4 a | 4.1 | 2.8 | 11.7 | 10.2 |
| F4 b | 3.3 | 2.7 | 5.0 | 6.4 |

These results predict that F3* has binding potential to the mu-receptor, but its binding affinity is slightly lower than that of fentanyl (table 7). The geometrical position of fentanyl and F3* in the binding pocket of the mu-receptor is similar (table 8).

### Mu-opioid receptor function in vitro:

We used transfected HEK-293 cells and cultured dorsal root ganglion (DRG) neurons to examine mu-agonist binding, GTPγS binding, cAMP formation and modulation of TRPV1 currents by F-Fentanyl according to F3*, selected as described above, and by control mu-agonists. Incubation of HEK-293 cells at different pH-values was initiated after the expression of mu-opioid receptors at the membrane surface was completed and stable. Affinity and number of mu-ligand (³H-DAMGO) binding sites were slightly (nonsignificantly) lower at pH 5.5 compared to pH 7.5 (Tab. 9). Preincubation with the adenylyl cyclase activator for-skolin (FSK; 10 µM) and the phosphodiesterase inhibitor 3-isobotyl-1-methylxanthin (IBMX; 2 mM) (control groups representing 100 % cAMP accumulation compared to unstimulated cells) yielded no significant differences between pH values of 5.0, 6.0, 7.0 and 7.5 (Fig. 5). However, suppression of FSK/IBMX-stimulated cAMP formation by DAMGO and morphine was markedly stronger at pH 5.0 than at pH 7.5 (Tab. 9), indicating a higher efficacy of mu-agonist-induced inhibition of adenylyl cyclase activity in acidic conditions.

| Tab. 9. Wild-type mu-receptors: mu-agonist binding and cAMP inhibition. | | | | | |
|---|---|---|---|---|---|
| | ³H-DAMGO | ³H-DAMGO | | 10 µM DAMGO | 10 µM Morphine |
| pH | K_{d} (nM) | Bₘₐₓ (fmol/mg) | pH | cAMP (% baseline) | |
| 5,5 | 5,561 | 14,04 | 5,0 | 43 ± 6 | 46 ± 11 |
| 6,5 | 2,101 | 15,50 | 6,5 | 68 ± 9 | 64 ± 13 |
| 7,5 | 1,149 | 23,95 | 7,5 | 82 ± 7 | 80 ± 10 |

Using fluoro-fentanyl (F-fentanyl) or fentanyl as mu-opioid receptor ligands the following results were obtained: Competitive binding experiments were performed using a fixed concentration of ³H-DAMGO (4 nM) in the presence of increasing concentrations (10⁻⁶ - 1 µM) of unlabelled F-fentanyl and the half maximal inhibitory concentration (IC₅₀) was calculated. The resulting IC₅₀ values were 1.03 nM (pH 5.5), 4.78 nM (pH 6.5) and 139.7 nM (pH 7.5), indicating that F-fentanyl displayed increasing affinity to mu-receptors with decreasing pH values. Thus, the lower the IC₅₀, the higher is the affinity of F-fentanyl to the mu-receptor (see also Fig. 5).

Both F-fentanyl and fentanyl produced significantly stronger reductions of FSK/IBMX-stimulated cAMP at pH 5.0 and 6.0 compared to pH 7.5 (Fig. 6). The difference between cAMP values at pH 5.0 and pH 7.5 seemed to be more pronounced for F-fentanyl than for fentanyl (F-fentanyl: **p=0.0011; fentanyl: *p=0.0118; ANOVA; Fig. 6). This demonstrates that F-fentanyl activates mu-opioid receptors predominantly during acidic conditions and is more effective in binding and receptor activation than fentanyl.

We also assessed G-protein activation following mu-receptor activation by 35S-GTPyS-binding. F-fentanyl yielded half maximal effective concentrations (EC50) of 0.25 nM (pH 5.5) and 4.14 nM (pH 7.5). The corresponding values for fentanyl were 53.37 nM (pH 5.5) and 41.91 nM (pH 7.5). Thus, F-fentanyl was functionally more potent at pH 5.5 than at pH 7.5, and it was more potent than fentanyl at all pH values.

### Antinociceptive effects of opioid ligands in vivo

In rats with unilateral hindpaw inflammation induced by intraplantar (i.pl.) complete Freund's adjuvant (CFA), fentanyl and F-fentanyl were injected i.pl. into the inflamed paw. At doses up to 1 µg, both drugs produced dose-dependent paw pressure threshold (PPT) elevations (antinociceptive effects) in the inflamed but not in the contralateral noninflamed paw. The effects of F-fentanyl were slightly stronger (Fig. 7). Consistent with our previous studies, this indicates that both drugs activate peripheral opioid receptors in inflamed but not in noninflamed tissue (Fig. 7). At higher doses fentanyl produced effects also on the contralateral paw, suggesting that the drug was absorbed into the circulation and produced central antinociceptive effects (Fig. 7). This was not the case for F-fentanyl (Fig. 7), indicating that, even if the drug reached the CNS after absorption into the circulation, it did not activate central opioid receptors. The intravenous (i.v.) injection of high doses (32 µg/kg) was lethal in the case of fentanyl (due to central respiratory depression). The same i.v. dose of F-fentanyl did not elicit any noticeable central effects but produced selective antinociception in the inflamed paw (Fig. 8). This indicates that peripheral opioid receptors in the injured tissue but no central receptors were activated.

### Methods used in the examples of the present invention

Details regarding software and computer-based methods are provided in the examples of the present invention
Cell culture and transfection: HEK293 cells are maintained in DMEM media supplemented with 10% fetal bovine serum and 1% streptomycin-penicillin, in 5 % CO2 at 37°C. Cells are passaged every 2-4 days and are not used above passage number 30. HEK293 cells are plated on poly-lysine-coated 96 well plates for ELISA and on 100 mm diameter plastic culture dishes for binding studies. After 2 days HEK293 cells are transiently transfected with 12 µg (binding experiments) and 0.1 µg (ELISA experiments) cDNAs of rat wildtype or mutant mu-opioid receptor using FuGENE 6 Transfection Reagent (Roche Diagnostics). Twenty four h after transfection, cells are preincubated at room temperature for 20 min at varying pH values (5.5-7.4) and then processed for further experiments.
Cyclic AMP enzyme linked immunosorbent assay (ELISA): Transfected cells are cultivated in a 96-well-plate and incubated with 10 µM forskolin, 2 mM 3-Isobutyl-1-methyl-Xanthin (IBMX) in the absence or presence of morphine/DAMGO under different pH conditions (5.0; 5.5; 6.0; 6.5; 7.0; 7.4) for min 20 min at 37° C. cAMP measurements are performed using the cAMP Biotrak Enzymeimmunoassay System (Amersham Biosciences) protocol following the manufacturer's instructions. Non-bound cAMP peroxidase oxidizes tetramethylbenzidine to a blue derivate. The reaction is stopped by applying sulphuric acid resulting in the accumulation of a yellow dye. The intensity of the color is detected with an ELISA-photometer at 450 nm.
Ligand binding: Membranes of HEK 293 cells expressing mu-opioid receptors are washed twice with 10 ml of Tris buffer (Trizma Preset Crystals pH 7.4; Sigma), harvested with a scraper, homogenized and centrifuged at 42000 g and 4°C for 20 min. The pellet is homogenized in 10 ml Tris and centrifuged at 42000g and 4°C for 20 min. Protein concentration is determined using the Bradford method. Appropriate concentrations of cell membranes (200 µg) are incubated in a final volume of 400 µl Tris buffer (pH 5.5, 6.5 or 7.4) with increasing concentrations of e.g. 3H-DAMGO (0.5 nM - 16 nM) (51Ci/mmol; Amersham) (or other ligands) in the absence and presence of 10 µM unlabelled naloxone. The presence of naloxone defines non-specific binding, which typically represents 15 to 35 % of total binding. Filters are soaked in 0.1 % (w/v) polyethyleneimine solution for 15 min before using. Bound and free ligand are separated by rapid filtration under vacuum through Whatman GF/B glass fiber filters, followed by four washes with cold Tris buffer. Bound radioactivity is determined by liquid scintillation spectrophotometry at 69% counting efficiency for 3H after overnight extraction of the filters in 3 ml of scintillation fluid.
Guanosine-5'-O-(3-³⁵S-thio)-triphosphate (³⁵S-GTPγ_{S}) binding: After preincubation for 20 min at varying pH values (5.5-7.4), HEK 293 cells expressing wild-type or mutant opioid receptors are washed two times with 10 ml PBS, harvested with a scraper in 10 ml ³⁵S-GTPγS assay buffer (50 mM Tris-HCL, 5 mM MgCl₂, 0.2 mM EGTA, 100 mM NaCl, and 1 mM dithiothreitol), homogenized and centrifuged at 42000 g and 4°C for 10 min. Cell pellets are resuspended in 10 ml ³⁵S-GTPγS assay buffer, homogenized and centrifuged again. Protein concentration is measured using the Bradford method. Adequate concentrations of protein (10-50 µg), varying concentrations of DAMGO (10⁻¹² 10 ⁻⁴ M) (or other ligands), 50 µM GDP and 0.05 nM ³⁵S-GTPγS in a total volume of 800 µl are incubated for 2 h to generate concentration-effect curves. Basal binding is detected in the absence of agonist and non-specific binding in the presence of 10 µM cold GTPγS. Bound and free ³⁵S-GTPγS are separated by vacuum filtration through GF/B filters. Quantification of bound ³⁵S-GTPγS is achieved by a liquid scintillation counter. Similar to our previous studies, saturation analysis of agonist-stimulated GTPγS binding is performed to determine the K_{d} of GTPγS and the total amount (Bₘₐₓ) of G-protein binding to the opioid receptor. Similar experiments are performed in DRG membranes obtained from the inflamed or noninflamed limbs of animals treated with intraplantar CFA, as commonly carried out in the art.

## Claims

1. Method for identifying compounds that bind a target opioid receptor in a pH-dependent manner, comprising
a) modification of a base compound to produce a modified compound,
b) determination of the acid dissociation constant (pKa) of said modified compound,
c) selection of said modified compound for further processing when pKa of said modified compound is less than 7, preferably between 4 and 7, more preferably between 5 and 7, and
d) testing the binding of the modified compound in said target opioid receptor,
wherein the modified compound binds the target opioid receptor at a pH value below 7, and/or
wherein steps a) to d) are simulations carried out by one or more computer programmes, and wherein step d) is carried out with the modified compound and the base compound, successively, in any order, using computer implemented methods in a three-dimensional (3D) binding pocket and/or receptor activation path model, wherein a modified compound is identified as binding a target opioid receptor in a pH-dependent manner when the binding of said target opioid receptor by the modified compound in step d) is improved or the same in comparison to the binding of said target opioid receptor by the base compound.

2. Method according to claim 1 for identifying compounds that bind and activate a target opioid receptor in a pH-dependent manner, whereby step d) of claim 1 comprises testing the binding of the modified compound to, and activation of said target opioid receptor.

3. Method according to any one of the preceding claims, **characterised in that** the simulations carried out by one or more computer programmes of steps a) to d) are carried out as individual software modules combined to be automatically carried out on a computing device.

4. Method according to any one of the preceding claims, **characterized in that** the method additionally comprises identifying chemical residues (e.g. amino acids and others) within the receptor that are responsible for pH-dependent binding and/or activation of the receptor.

5. Method according to any one of the preceding claims, **characterised in that** the identified modified and/or base compound is chemically synthesized.

6. Method according to any one of the preceding claims, **characterized in that** the chemically synthesized modified compound and/or base compound is tested for opioid receptor binding using in vitro methods.

7. Method according to the preceding claim, **characterized in that** the chemically synthesized modified compound and/or base compound is tested for opioid receptor activation (agonist function) using in vitro methods.

8. Method according to any one of the preceding claims, **characterized in that** the base compound is an opioid or opioid derivative or analogue, which exhibits a nitrogen atom that can be protonated or non-protonated.

9. Method according to the preceding claim, wherein protonation of said nitrogen atom occurs in modified compounds with a pKa of less than 7 when at pH values less than 7.

10. Method according to any one of the preceding claims, **characterised in that** the target opioid receptor is selected from the group consisting of the mu-receptor, delta-receptor, kappa-receptor and/or subtypes thereof and/or the target receptor is of the family of opioid-related G-protein coupled receptors such as the rhodopsin, opsin, chemokine, cannabinoid, dopamine, histamine, muscarinic, neurotensin, adrenergic, adenosine and/or any other opioid-related receptor.

11. Method according to any one of the preceding claims, **characterised in that** the modification of the base compound is replacement of one or more hydrogen atoms with one or more atoms of greater electronegativity compared to hydrogen.

12. Method according to the preceding claim, wherein the atom of greater electronegativity compared to hydrogen is a halogen.

13. Method according to the preceding claim, wherein the halogen is a fluorine atom.

14. Method according to any one of the preceding claims, **characterised in that** the evaluation of binding of and/or receptor activation by the modified or base compound in the binding pocket model and/or receptor activation path model is carried out using steric and/or energetic criteria measured and/or determined during simulation of binding and/or receptor activation.

## Patentansprüche

1. Verfahren zur Identifikation von Verbindungen, die einen Zielopioidrezeptor abhängig vom pH-Wert binden, umfassend
a) Modifikation einer Basenverbindung, um eine modifizierte Verbindungen zu erzeugen,
b) Bestimmung der Säuredissoziationskonstanten (pKa) der modifizierten Verbindung,
c) Auswahl der modifizierten Verbindung für das weitere Verfahren, wenn der pKa-Wert der modifizierten Verbindung niedriger als 7 ist, bevorzugt zwischen 4 und 7, bevorzugter zwischen 5 und 7, und
d) Testen der Bindung der modifizierten Verbindung in dem Zielopioidrezeptor,
wobei die modifizierte Verbindung den Zielopioidrezeptor bei einem pH-Wert unter 7 bindet, und/oder wobei die Schritte a) bis d) Simulationen sind, die durch ein oder mehrere Computerprogramme ausgeführt werden, und wobei Schritt d) mit der modifizierten Verbindung und der Basenverbindung der Reihe nach in einer beliebigen Reihenfolge durchgeführt wird, wobei auf einem Computer implementierte Verfahren in einer dreidimensionalen (3D) Bindungstasche und/oder einem Rezeptoraktivierungspfadmodell angewendet werden, wobei eine modifizierte Verbindung als einen Zielopioidrezeptor abhängig vom pH-Wert bindend identifiziert wird, wenn die Bindung des Zielopioidrezeptors durch die modifizierte Verbindung in Schritt d) im Vergleich zur Bindung des Zielopioidrezeptors durch die Basenverbindung verbessert oder dieselbe ist.

2. Verfahren nach Anspruch 1 zur Identifizierung von Verbindungen, die einen Zielopioidrezeptor abhängig vom pH-Wert binden und aktivieren, wobei Schritt d) nach Anspruch 1 das Testen der Bindung der modifizierten Verbindung an den Zielopioidrezeptor und die Aktivierung des Zielopioidrezeptors umfasst.

3. Verfahren nach einem der vorhergehenden Ansprüche, **dadurch gekennzeichnet, dass** die Simulationen, die durch ein oder mehrere Computerprogramme von Schritt a) bis d) ausgeführt werden, als einzelne Softwaremodule durchgeführt werden, die kombiniert sind, um automatisch auf einem Computer ausgeführt zu werden.

4. Verfahren nach einem der vorhergehenden Ansprüche, **dadurch gekennzeichnet, dass** das Verfahren zusätzlich die Identifikation von chemischen Resten (z. B. Aminosäuren und anderen) innerhalb des Rezeptors umfasst, die für eine pH-Wert-abhängige Bindung und/oder Aktivierung des Rezeptors verantwortlich sind.

5. Verfahren nach einem der vorhergehenden Ansprüche, **dadurch gekennzeichnet, dass** die identifizierte, modifizierte Verbindung und/oder Basenverbindung chemisch synthetisiert wird.

6. Verfahren nach einem der vorhergehenden Ansprüche, **dadurch gekennzeichnet, dass** die chemisch synthetisierte, modifizierte Verbindung und/oder Basenverbindung für die Bindung von Opioidrezeptoren getestet wird, wobei In-vitro-Verfahren angewendet werden.

7. Verfahren nach dem vorhergehenden Anspruch, **dadurch gekennzeichnet, dass** die chemisch synthetisierte, modifizierte Verbindung und/oder Basenverbindung für die Aktivierung von Opioidrezeptoren (Agonistfunktion) getestet wird, wobei In-vitro-Verfahren angewendet werden.

8. Verfahren nach einem der vorhergehenden Ansprüche, **dadurch gekennzeichnet, dass** die Basenverbindung ein Opioid, ein Opioidderivat oder -analogon ist, das ein Stickstoffatom aufweist, das protoniert oder nicht protoniert sein kann.

9. Verfahren nach dem vorhergehenden Anspruch, wobei die Protonierung des Stickstoffatoms in modifizierten Verbindungen mit einem pKa-Wert von unter 7 stattfindet, wenn der pH-Wert unter 7 ist.

10. Verfahren nach einem der vorhergehenden Ansprüche, **dadurch gekennzeichnet, dass** der Zielopioidrezeptor aus der Gruppe, die aus dem mu-Rezeptor, delta-Rezeptor, kappa-Rezeptor und/oder Unterarten davon besteht, ausgewählt wird, und/oder der Zielrezeptor zur Familie der opioidverwandten G-Proteingekoppelten Rezeptoren gehört, wie der Rhodopsin-, Opsin-, Chemokin-, Cannabinoid-, Dopamin-, Histamin-, muscarinische, Neurotensin-, adrenerge, Adenosin- und/oder jeglicher anderer opioidverwandter Rezeptor.

11. Verfahren nach einem der vorhergehenden Ansprüche, **dadurch gekennzeichnet, dass** die Modifikation der Basenverbindung ein Austausch von einem oder mehreren Wasserstoffatomen mit einem oder mehreren Atomen mit höherer Elektronegativität im Vergleich zu Wasserstoff ist.

12. Verfahren nach dem vorhergehenden Anspruch, wobei das Atom, das eine höhere Elektronegativität im Vergleich zum Wasserstoff aufweist, ein Halogen ist.

13. Verfahren nach dem vorhergehenden Anspruch, wobei das Halogen ein Fluoratom ist.

14. Verfahren nach einem der vorhergehenden Ansprüche, **dadurch gekennzeichnet, dass** die Bewertung der Bindung von und/oder Rezeptoraktivierung durch eine(r) modifizierte(n) Verbindung oder Basenverbindung in dem Bindungstaschenmodell und/oder Rezeptoraktivierungspfadmodell durch Anwenden von sterischen und/oder energetischen Kriterien, die während der Simulation der Bindung und/oder Rezeptoraktivierung gemessen und/oder bestimmt wurden, durchgeführt wird.

## Revendications

1. Procédé pour identifier des composés liant un récepteur opioïde cible de manière dépendante du pH, qui comprend
a) la modification d'un composé de base pour produire un composé modifié,
b) la détermination de la constante de dissociation acide (pKa) dudit composé modifié,
c) la sélection dudit composé modifié pour traitement ultérieur lorsque la pKa dudit composé modifié est inférieure à 7, de préférence entre 4 et 7, de manière davantage préférée entre 5 et 7, et
d) le test de la liaison du composé modifié dans ledit récepteur opioïde cible,
dans lequel le composé modifié lie le récepteur opioïde cible à une valeur de pH inférieure à 7, et/ou
dans lequel les étapes a) à d) sont des simulations réalisées par un ou plusieurs programmes informatiques, et dans lequel l'étape d) est réalisée avec le composé modifié et le composé de base, successivement, dans n'importe quel ordre, en utilisant des procédés mis en oeuvre par ordinateur, dans un modèle de poche de liaison et/ou d'activation de récepteur en trois dimensions (3D),
dans lequel un composé modifié est identifié comme liant un récepteur opioïde cible de manière dépendante du pH lorsque la liaison dudit récepteur opioïde cible par le composé modifié dans l'étape d) est amélioré ou identique en comparaison avec la liaison dudit récepteur opioïde cible par le composé de base.

2. Procédé selon la revendication 1 pour identifier des composés liant et activant un récepteur opioïde cible de manière dépendante du pH, moyennant quoi l'étape d) de la revendication 1 comprend le test de la liaison du composé modifié audit récepteur opioïde cible et l'activation de celui-ci.

3. Procédé selon l'une quelconque des revendications précédentes, **caractérisé en ce que** les simulations réalisées par un ou plusieurs programmes informatiques des étapes a) à d) sont réalisées comme des modules logiciels individuels combinés pour être réalisés de manière automatique sur un dispositif informatique.

4. Procédé selon l'une quelconque des revendications précédentes, **caractérisé en ce que** le procédé comprend en outre l'identification de résidus chimiques (par exemple, des acides aminés et autres) au sein du récepteur qui sont responsables de la liaison dépendante du pH et/ou de l'activation du récepteur.

5. Procédé selon l'une quelconque des revendications précédentes, **caractérisé en ce que** le composé modifié et/ou de base identifié est synthétisé chimiquement.

6. Procédé selon l'une quelconque des revendications précédentes, **caractérisé en ce que** le composé modifié et/ou le composé de base synthétisé chimiquement est testé pour la liaison au récepteur opioïde en utilisant des procédés in vitro.

7. Procédé selon la revendication précédente, **caractérisé en ce que** le composé modifié et/ou le composé de base synthétisé chimiquement est testé pour l'activation du récepteur opioïde (fonction agoniste) en utilisant des procédés in vitro.

8. Procédé selon l'une quelconque des revendications précédentes, **caractérisé en ce que** le composé de base est un opioïde ou un dérivé opioïde ou analogue, qui présente un atome d'azote qui peut être protoné ou non-protoné.

9. Procédé selon la revendication précédente, dans lequel la protonation dudit atome d'azote survient dans des composés modifiés ayant une pKa inférieure à 7 lorsqu'à des valeurs de pH inférieures à 7.

10. Procédé selon l'une quelconque des revendications précédentes, **caractérisé en ce que** le récepteur opioïde cible est sélectionné dans le groupe constitué du récepteur mu, du récepteur delta, du récepteur kappa et/ou de sous-types de ceux-ci et/ou le récepteur cible est de la famille des récepteurs couplés aux protéines G liés aux opioïdes tels que la rhodopsine, l'opsine, la chimiokine, le cannabinoïde, la dopamine, l'histamine, le récepteur muscarinique, la neurotensine, le récepteur adrénergique, l'adénosine et/ou tout autre récepteur lié aux opioïdes.

11. Procédé selon l'une quelconque des revendications précédentes, **caractérisé en ce que** la modification du composé de base est le remplacement d'un ou plusieurs atomes d'hydrogène par un ou plusieurs atomes de plus grande électronégativité par rapport à l'hydrogène.

12. Procédé selon la revendication précédente, dans lequel l'atome de plus grande électronégativité par rapport à l'hydrogène est un halogène.

13. Procédé selon la revendication précédente, dans lequel l'halogène est un atome de fluor.

14. Procédé selon l'une quelconque des revendications précédentes, **caractérisé en ce que** l'évaluation de la liaison du composé modifié ou de base et/ou l'activation du récepteur par celui-ci dans le modèle de poche de liaison et/ou le modèle d'activation du récepteur est réalisée en utilisant des critères stériques et/ou énergétiques mesurés et/ou déterminés durant la simulation de la liaison et/ou de l'activation du récepteur.
